# EUROPEAN PATENT SPECIFICATION

(11) **EP 2 214 840 B1**
(45) Date of publication and mention of the grant of the patent: **06.05.2015**
(21) Application number: 08832781.2
(22) Date of filing: 30.10.2008
(51) Int. Cl.: A61K 39/02

(54) **COMPOSITIONS AND METHODS OF ENHANCING IMMUNE RESPONSES TO FLAGELLATED BACTERIUM**
ZUSAMMENSETZUNGEN UND VERFAHREN ZUR VERBESSERUNG DER IMMUNREAKTION AUF FLAGELLATBAKTERIEN
COMPOSITIONS ET PROCÉDÉS POUR AMÉLIORER LES RÉPONSES IMMUNITAIRES DIRIGÉES CONTRE UNE BACTÉRIE FLAGELLÉE

(30) Priority: 30.10.2007 US 983803 P
(43) Date of publication of application: 11.08.2010
(73) Proprietor: THE BOARD OF TRUSTEES OF THE UNIVERSITY OF ARKANSAS, Little Rock AR 72207 (US); THE TEXAS A&M UNIVERSITY SYSTEM, College Station, Texas 77843-3369 (US)
(72) Inventor: BOTTJE, Walter, Fayetteville AR 72703 (US); HARGIS, Billy, Fayetteville AR 72703 (US); BERGHMAN, Luc, College Station TX 77845 (US); KWON, Young, Min, West Fork AR 72774 (US); COLE, Kimberly, Raymond OH 43067 (US); COX, Mandy, Fayetteville AR 72701 (US); LAYTON, Sherryll, Rogers, AR 72758 (US)
(74) Representative: Kremer, Simon Mark
(86) International application number: PCT/US2008/081813
(87) International publication number: WO 2009/059018

(56) References cited:
- WO-A2-2007/103048
- US-A- 4 420 497
- US-A- 4 510 674
- US-A- 5 800 632
- US-A1- 2006 249 202
- US-A1- 2007 227 586
- US-B1- 7 098 058
- KAJIKAWA ET AL: "Intragastric immunization with recombinant Lactobacillus casei expressing flagellar antigen confers antibody-independent protective immunity against Salmonella enterica serovar Enteritidis", VACCINE, ELSEVIER LTD, GB, vol. 25, no. 18, 17 April 2007 (2007-04-17), pages 3599-3605, XP022032569, ISSN: 0264-410X, DOI: 10.1016/J.VACCINE.2007.01.055
- COX MANDY M ET AL: "Scarless and site-directed mutagenesis in Salmonella enteritidis chromosome", BMC BIOTECHNOLOGY, BIOMED CENTRAL LTD. LONDON, GB, vol. 7, no. 59, 17 September 2007 (2007-09-17), pages 1-10, XP002558959, ISSN: 1472-6750, DOI: 10.1186/1472-6750-7-59

### SEQUENCE LISTING

<110> **THE BOARD OF TRUSTEES OF THE UNIVERSITY OF ARKANSAS**
<120> **COMPOSITIONS AND METHODS OF ENHANCING IMMUNE RESPONSES TO FLAGELLATED BACTERIUM**
<130> 013961-9037-WO00
<140> New International Application
   <141> 2008-10-30
<150> US 60/983,803
   <151> 2007-10-30
<160> 29
<170> PatentIn version 3.5
<210> 1
   <211> 7
   <212> PRT
   <213> Salmonella enteritidis
<400> 1
   Ser Ala Ile Thr Asn Leu Gly
   1               5
<210> 2
   <211> 7
   <212> PRT
   <213> Escherichia coli
<400> 2
   Ser Ala Val Thr Asn Leu Asn
   1               5
<210> 3
   <211> 11
   <212> PRT
   <213> Homo sapiens
<400> 3
   Trp Ala Glu Lys Gly Tyr Tyr Thr Met Ser Cys
   1               5                   10
<210> 4
   <211> 11
   <212> PRT
   <213> Gallus gallus
<400> 4
   Trp Met Thr Thr Ser Tyr Ala Pro Thr Ser Ser
   1               5                   10
<210> 5
   <211> 10
   <212> PRT
   <213> Anas sp.
<400> 5
   Trp Asn Lys Thr Ser Tyr Ala Pro Met Asn
   1               5                   10
<210> 6
   <211> 10
   <212> PRT
   <213> Mus sp.
<400> 6
   Trp Ala Lys Lys Gly Tyr Tyr Thr Met Lys
   1               5                   10
<210> 7
   <211> 10
   <212> PRT
   <213> Bos taurus
<400> 7
   Trp Ala Pro Lys Gly Tyr Tyr Thr Leu Ser
   1               5                   10
<210> 8
   <211> 261
   <212> PRT
   <213> Homo sapiens
<400> 8
<210> 9
   <211> 272
   <212> PRT
   <213> Gallus gallus
<400> 9
   Met Asn Glu Ala Tyr Ser Pro Ala Ala Pro Arg Pro Met Gly Ser Thr
   1               5                   10                  15
   Ser Pro Ser Thr Met Lys Met Phe Met Cys Phe Leu Ser Val Phe Met
               20                  25                  30
   Val Val Gln Thr Ile Gly Thr Val Leu Phe Cys Leu Tyr Leu His Met
           35                  40                  45
   Lys Met Asp Lys Met Glu Glu Val Leu Ser Leu Asn Glu Asp Tyr Ile
       50                  55                  60
   Phe Leu Arg Lys Val Gln Lys Cys Gln Thr Gly Glu Asp Gln Lys Ser
   65 70 75 80
   Thr Leu Leu Asp Cys Glu Lys Val Leu Lys Gly Phe Gln Asp Leu Gln
   85 90 95
   Cys Lys Asp Arg Thr Ala Ser Glu Glu Leu Pro Lys Phe Glu Met His
   100 105 110
   Arg Gly His Glu His Pro His Leu Lys Ser Arg Asn Glu Thr Ser Val
   115 120 125
   Ala Glu Glu Lys Arg Gln Pro Ile Ala Thr His Leu Ala Gly Val Lys
   130 135 140
   Ser Asn Thr Thr Val Arg Val Leu Lys Trp Met Thr Thr Ser Tyr Ala
   145 150 155 160
   Pro Thr Ser Ser Leu Ile Ser Tyr His Glu Gly Lys Leu Lys Val Glu
   165 170 175
   Lys Ala Gly Leu Tyr Tyr Ile Tyr Ser Gln Val Ser Phe Cys Thr Lys
   180 185 190
   Ala Ala Ala Ser Ala Pro Phe Thr Leu Tyr Ile Tyr Leu Tyr Leu Pro
   195 200 205
   Met Glu Glu Asp Arg Leu Leu Met Lys Gly Leu Asp Thr His Ser Thr
   210 215 220
   Ser Thr Ala Leu Cys Glu Leu Gln Ser Ile Arg Glu Gly Gly Val Phe
   225 230 235 240
   Glu Leu Arg Gln Gly Asp Met Val Phe Val Asn Val Thr Asp Ser Thr
   245 250 255
   Ala Val Asn Val Asn Pro Gly Asn Thr Tyr Phe Gly Met Phe Lys Leu
   260 265 270
<210> 10
   <211> 15
   <212> PRT
   <213> Salmonella enteritidis
<400> 10
   Val Gln Asn Arg Phe Asn Ser Ala Ile Thr Asn Gly Leu Asn Thr
   1 5 10 15
<210> 11
   <211> 15
   <212> PRT
   <213> Salmonella enteritidis
<400> 11
   Gly Ala Val Gln Asn Arg Phe Asn Ser Ala Ile Thr Leu Asn Gly
   1 5 10 15
<210> 12
   <211> 13
   <212> PRT
   <213> Salmonella enteritidis
<400> 12
   Ile Gln Asn Arg Phe Asp Ser Ala Ile Thr Leu Asn Gly
   1 5 10
<210> 13
   <211> 18
   <212> DNA
   <213> Artificial Sequence
<220>
   <223> I-SceI enzyme recognition sequence
<400> 13
   tagggataac agggtaat 18
<210> 14
   <211> 20
   <212> DNA
   <213> Artificial Sequence
<220>
   <223> loop 9 up
<400> 14
   tgtacaagtg gacgccaatc 20
<210> 15
   <211> 24
   <212> DNA
   <213> Artificial Sequence
<220>
   <223> loop 9 up
<400> 15
   gttatcgccg tctttgatat agcc 24
<210> 16
   <211> 20
   <212> DNA
   <213> Artificial Sequence
<220>
   <223> loop 9 down
<400> 16
   atttcccgtt atgccgcagc 20
<210> 17
   <211> 20
   <212> DNA
   <213> Artificial Sequence
<220>
   <223> loop 9 down
<400> 17
   gttaaacaga gggcgacgag 20
<210> 18
   <211> 68
   <212> DNA
   <213> Artificial Sequence
<220>
   <223> I-SceI/Kmr gene
<400> 18
   gctatatcaa agacggcgat aactaactat aacggtccta aggtagcgaa tttccgggga 60
tccgtcga 68
<210> 19
   <211> 40
   <212> DNA
   <213> Artificial Sequence
<220>
   <223> I-SceI/Kmr gene
<400> 19
   gctgcggcat aacgggaaat tgtaggctgg agctgcttcg 40
<210> 20
   <211> 20
   <212> DNA
   <213> Artificial Sequence
<220>
   <223> inside Kmr gene: sequencing
<400> 20
   caaaagcgct ctgaagttcc 20
<210> 21
   <211> 20
   <212> DNA
   <213> Artificial Sequence
<220>
   <223> inside Kmr gene: sequencing
<400> 21
   gcgtgagggg atcttgaagt 20
<210> 22
   <211> 44
   <212> DNA
   <213> Artificial Sequence
<220>
   <223> fliC/ loop 9 up
<400> 22
   cggttctgta cggaggagga gttatcgccg tctttgatat agcc 44
<210> 23
   <211> 83
   <212> DNA
   <213> Artificial Sequence
<220>
   <223> fliC/ loop 9 down
<400> 23
   tcctcctccg tacagaaccg tttcaactcc gctattacca acctgggcaa cacctcctcc 60
   tccatttccc gttatgccgc agc 83
<210> 24
   <211> 81
   <212> DNA
   <213> Artificial Sequence
<220>
   <223> fliC-hCD154/ loop 9 up
<400> 24
   ggaggtgttg cccaggttgg taatagcgga gttgaaacgg ttctgtacgg aggaggagtt 60
   atcgccgtct ttgatatagc c 81
<210> 25
   <211> 85
   <212> DNA
   <213> Artificial Sequence
<220>
   <223> fliC-hCD154/ loop 9 down
<400> 25
   ccaacctggg caacacctcc tcctcctggg cagaaaaagg ttattatacc atgtcttcct 60
   cctccatttc ccgttatgcc gcagc 85
<210> 26
   <211> 81
   <212> DNA
   <213> Artificial Sequence
<220>
   <223> fliC-cCD154/ loop 9 up
<400> 26
   ggaggtgttg cccaggttgg taatagcgga gttgaaacgg ttctgtacgg aggaggagtt 60
   atcgccgtct ttgatatagc c 81
<210> 27
   <211> 85
   <212> DNA
   <213> Artificial Sequence
<220>
   <223> fliC-cCD154/ loop 9 down
<400> 27
   ccaacctggg caacacctcc tcctcctgga tgaccacctc ctatgcgccg acctcctcct 60
   cctccatttc ccgttatgcc gcagc 85
<210> 28
   <211> 20
   <212> DNA
   <213> Artificial Sequence
<220>
   <223> outer regions of loop 9: sequencing
<400> 28
   gccatctcgc ttggtgataa 20
<210> 29
   <211> 20
   <212> DNA
   <213> Artificial Sequence
<220>
   <223> outer regions of loop 9: sequencing
<400> 29
   cgctggtatt ttgcggtaca 20

## Description

### INTRODUCTION

*Salmonella,* continues to be one of the most commonly reported bacterial causes of human food-borne infections worldwide, and epidemiological evidence indicates that poultry and poultry products are a significant source of human infection. In the United States, an estimated 1.4 million cases of human *Salmonellosis* are reported annually. Of these cases, *S. enterica* serovars Enteritidis (SE) and Typhimurium (ST) are the most commonly isolated, although a number of other serovars have also been shown to cause enteritis in humans.

*Salmonella* infrequently causes apparent clinical disease in poultry flocks. However, infection in young chicks with some *Salmonella* isolates results in 2% mortality within the first 48 hours post-hatch, and up to 20% morbidity within the first five days. Therefore, increasing the resistance of the poultry population against *Salmonella* will not only reduce the impact of low level disease on performance, but will also reduce the significant health risk for the human population as well.

KAJIKAWA ET AL., VACCINE, vol. 25, no. 18, 17 April 2007, pages 3599-3605, discloses a recombinant *Lactobacillus casei* expressing a flagellar antigen from *Salmonella enterica* serovar Enteritidis. Intragastric immunization of the recombinant strain conferred protective immunity against *Salmonella* infection in mice.

COX MANDY M ET AL., BMC BIOTECHNOLOGY, vol. 7, no. 59, 17 September 2007, pages 1 -10, discloses insertion of a section of the M2e epitope (LM2) of Influenza A virus, a domain of CD154 (CD154s) or a combination of both into the outer membrane protein LamB of *S.enteritidis.*

WO 2007/103048 A2 discloses fusion proteins and DNA conjugates which contain a TLR/CD40/ agonist and optional antigen combination.

### SUMMARY

The present invention is as defined in the claims.

A bacterial vaccine comprising a *fliC* polynucleotide sequence encoding a first polypeptide and a second polynucleotide sequence encoding a CD 154 polypeptide is disclosed. The CD 154 polypeptide is capable of binding CD40 and has fewer than 50 amino acids, and includes amino acids 140-149 of SEQ ID NO: 8 or at least one of SEQ ID NO's 3, 4,5,6 & 7. The fliC polypeptide consists of SEQ ID NO: 1, 2, 10, 11 or 12, or an immunogenic fragment thereof. The fliC polynucleotide and the CD154 polynucleotide are inserted into a polynucleotide sequence encoding an external portion of a transmembrane protein.

A vaccine comprising a variant of *Salmonella enteritidis* 13A is also disclosed. The *Salmonella* comprises a first polynucleotide sequence encoding a *fliC* polypeptide.

Also described herein are, methods of enhancing the immune response against a flagellated bacterium in a subject by administering a bacterium. The bacterium includes a first polynucleotide sequence encoding a *fliC* polypeptide and a second polynucleotide sequence encoding a CD 154 polypeptide. The CD 154 polypeptide is capable of binding CD40 and has fewer than 50 amino acids, and includes amino acids 140-149 of SEQ ID NO: 8 or a homolog thereof. The bacterium is administered in an amount effective to enhance the immune response of the subject to the flagellated bacterium.

Also described herein are, methods of enhancing the immune response against a flagellated bacterium in a subject by administering a variant of *Salmonella enteritidis* 13A. The *Salmonella* includes a first polynucleotide sequence encoding a *fliC* polypeptide. The *Salmonella* is administered in an amount effective to enhance the immune response of the subject to the flagellated bacterium.

Also described herein are, methods of reducing morbidity associated with infection with a flagellated bacterium in a subject by administering a bacterium. The bacterium includes a first polynucleotide sequence encoding a *fliC* polypeptide and a second polynucleotide sequence encoding a CD 154 polypeptide. The CD 154 polypeptide is capable of binding CD40, and has fewer than 50 amino acids, and includes amino acids 140-149 of SEQ ID NO: 8 or a homolog thereof. The bacterium is administered in an amount effective to reduce the morbidity of the subject after infection with the flagellated bacterium.

Also described herein are, methods of reducing the morbidity of infection with a flagellated bacterium in a subject by administering a variant of *Salmonella enteritidis* 13A. The *Salmonella* includes a first polynucleotide sequence encoding *a fliC* polypeptide. The *Salmonella* is administered in an amount effective to reduce the morbidity of the subject after infection with the flagellated bacterium.

### BRIEF DESCRIPTION OF THE DRAWINGS

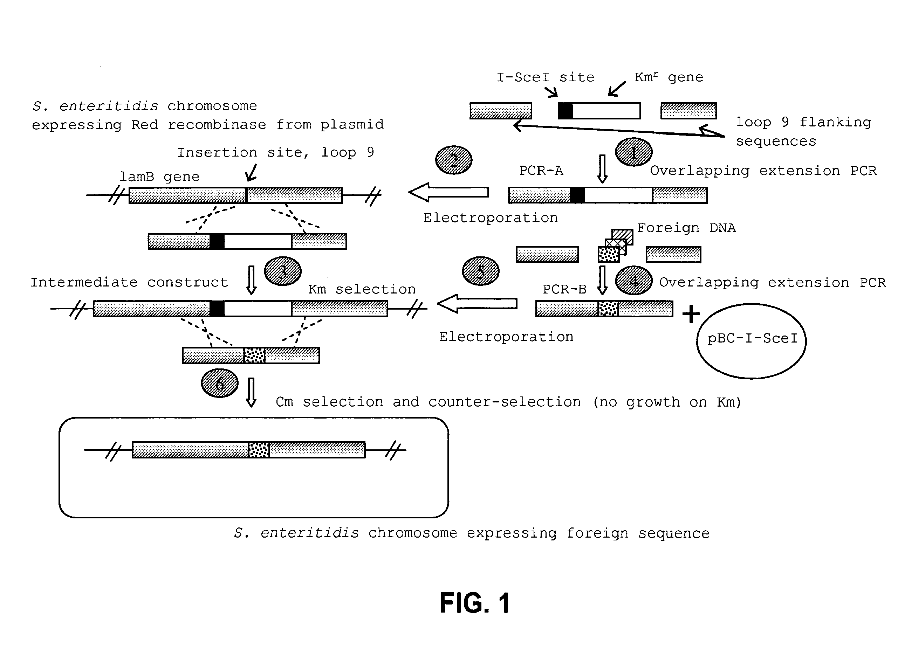

Figure 1 depicts the scheme for making site-directed mutations in *Salmonella enteritidis.*
Figure 2 depicts the design scheme of the overlapping extension PCR method used to generate the *fliC* and *fliC* -CD 154 insertions into loop 9 of the *lamB* polynucleotide.
Figure 3 is a bar graph showing the percent *Salmonella enteritidis* recovery as compared to the initial inoculum in the liver/spleen and cecal tonsils at 3 days post-inoculation with the indicated bacteria.
Figure 4 is a bar graph showing the percent *Salmonella enteritidis* recovery as compared to the initial inoculum in the liver/spleen and cecal tonsils at 21 days post-inoculation with the indicated bacteria.

### DETAILED DESCRIPTION

Vaccination against *Salmonella* is difficult because over 2,000 serovars have been described and immunity to one serovar generally does not confer immunity to a distinct serovar. Development of a vaccine to protect humans, poultry and other domesticated animals from *Salmonellosis* is needed. A vaccine capable of protecting against multiple serovars would be optimal. A vaccine comprising a highly conserved region of *fliC,* a flagellar filament protein found on flagellated Salmonella, is provided.

Recombinant DNA technologies enable relatively easy manipulation of many bacterial and viral species. Some bacteria and viruses are mildly pathogenic or non-pathogenic, but are capable of generating a robust immune response. These bacteria and viruses make attractive vaccine vectors for eliciting an immune response to antigens. Bacterial or viral vaccine vectors may mimic a natural infection and produce robust and long lasting immunity. Vaccine vectors are often relatively inexpensive to produce and administer. In addition, such vectors can often carry more than one antigen and may provide protection against multiple infectious agents.

In one aspect, a vaccine comprising a first polynucleotide sequence encoding *a fliC* polypeptide and a second polynucleotide sequence encoding a CD 154 polypeptide which is capable of binding CD40 is provided. Also described herein is the use of vaccine vectors, such as bacterial vectors, for vaccination and generation of immune responses against *Salmonella* and other flagellated pathogenic bacteria. *Salmonella* strains make suitable vaccine vectors because bacterial genes may be mutated or attenuated to create bacteria with low to no pathogenesis to the infected or immunized subject, while maintaining immunogenicity.

The majority of *Salmonella* isolates contain two genes that encode flagellar (H) antigens, *fliC* and *flj*B, which are alternately expressed by a phase-variation mechanism. The phase 1 antigens are encoded by *fliC* whereas *flj*B encodes the phase 2 antigens. A conserved region within *fliC* that has almost 100% homology between multiple *Salmonella* serovars and between *fliC* and *flj*B has been identified. This conserved region of *fliC* is depicted in SEQ ID NO: 1 and was used to generate several vaccine vectors as described in the Examples. Other possible polypeptides for use in vaccine vectors are disclosed in SEQ ID NO: 2 (a similar region of *fliC* from *E. coli*), SEQ ID NO: 10, SEQ ID NO: 11 or SEQ ID NO: 12. Immunogenic fragments of the *fliC* polypeptides may also be used to generate vaccines. In addition, there is extensive homology between this conserved region of *fliC* and flagellar sequences of other bacteria, such as *Shigella* and *E. coli,* such that a vaccine vector expressing *fliC* may enhance the immune response to flagellated bacteria generally. Therefore, expression of these protective epitopes on the surface of a *Salmonella* vaccine vector may induce protective immunity against multiple serovars of the organism and may allow immunization against all flagellated bacterium.

Involvement of dendritic cells (DCs) is essential for the initiation of a powerful immune response as they possess the unique ability to activate naïve T cells, causing T cell expansion and differentiation into effector cells. It is the role of the DC, which is an antigen presenting cell (APC) found in virtually all tissues of the body, to capture antigens, transport them to associated lymphoid tissue, and then present them to naive T cells. Upon activation by DCs, T cells expand, differentiate into effector cells, leave the secondary immune organs, and enter peripheral tissues. Activated cytotoxic T cells (CTLs) are able to destroy virus-infected cells, tumor cells or even APCs infected with intracellular parasites (e.g., *Salmonella*) and have been shown to be critical in the protection against viral infection.

CD40 is a member of the TNF-receptor family of molecules and is expressed on a variety of cell types, including professional antigen-presenting cells (APCs), such as DCs and B cells. Interaction of CD40 with its ligand CD 154 is extremely important and stimulatory for both humoral and cellular immunity. Stimulation of DCs via CD40, expressed on the surface of DCs, can be simulated by anti-CD40 antibodies. In the body, however, this occurs by interaction with the natural ligand for CD40 (i.e. CD154) expressed on the surface of activated T-cells. Interestingly, the CD40-binding regions of CD 154 have been identified. The CD40-binding region of CD 154 may be expressed on the surface of a vaccine vector, such as a *Salmonella* vaccine vector, and may result in an enhanced immune response against a co-presented peptide sequence.

*Salmonella* can survive the gastrointestinal tract of the host and give rise to a mucosal immune response. Oral vaccines using a *Salmonella* vector produce a robust mucosal immune response and are relatively easy to administer to both animals and humans. However, many of the current attenuated *Salmonella* vaccine strains are not as effective in generating a strong protective immune response as compared to their more virulent counterparts. Virulent strains provide a robust immune response but may also cause significant morbidity to the vaccinated subject. A *Salmonella* strain that could be used for effective mucosal, e.g., oral, vaccination would provide a vector that could be used to readily vaccinate a subject against one or more pathogenic agents, such as flagellated bacteria. Alternatively, a method of limiting an infection caused by a *Salmonella* vaccine vector would also be useful. Described herein are methods of limiting an infection caused by a *Salmonella* vaccine vector by administering the *Salmonella,* vaccine vector to a subject and administering a second vaccine vector comprising a first polynucleotide sequence encoding a *fliC* polypeptide. Administration of the second vaccine vector enhances the immune response to *Salmonella,* and limits the infection caused by the *Salmonella* vaccine vector. The second vaccine vector may be administered before, at the same time as or after the *Salmonella* vaccine vector.

A *Salmonella enteritidis* strain useful as a vaccine vector, and various recombinant vaccine vectors made using this strain, are described. Specifically, a *Salmonella enteritidis* 13A (SE13A) capable of expressing an exogenous *fliC* polypeptide is described. In addition, a vaccine vector and methods of enhancing an immune response in a subject by administering the vaccine vector comprising a first polynucleotide sequence encoding a *fliC* polypeptide and a second polynucleotide sequence encoding a polypeptide of CD154 or a homolog thereof that is capable of binding to CD40 are disclosed. The vaccine vectors may be used to enhance an immune response against *Salmonella* or another flagellated bacterium, such as *Escherichia coli* or *Shigella,* or may be used to reduce the morbidity associated with a flagellated bacterial infection.

A wild-type isolate of *Salmonella, Salmonella enteritidis* 13A (SE13A) (deposited with the American Type Culture Collection (ATCC) on September 13, 2006, deposit number PTA-7871), was selected based upon its unusual ability to cause mucosal colonization and submucosal translocation in chickens, permitting robust presentation of associated antigens or epitopes in commercial chickens. Importantly, this wild-type *Salmonella,* isolate causes no clinically detectable disease or loss of performance in commercial chickens, indicating little disease-causing potential of the wild-type *Salmonella* in vertebrate animals.

The SE13A isolate may be further attenuated by inactivating at least one gene necessary for sustained replication of the bacteria outside of laboratory or manufacturing conditions. Attenuated or variant *Salmonella* strains that can be used as vaccine vectors are described below. SE13A was used to generate attenuated *Salmonella* strains to develop vaccines and generate enhanced immune responses. SE13A is invasive, non-pathogenic for poultry and causes no measurable morbidity. These features result in an enhanced immune response as compared to non-invasive bacterial vectors. Attenuation of SE13A by mutation of genes that limit the ability of the bacterium to spread may increase the safety of the vaccine. For example, SE13A strains with mutations in *aroA* and/or *htrA* retain the ability to generate an immune response, but have limited replication in the host. Thus, the attenuation increases the safety of the vaccine vector without compromising the immunogenicity.

Mutations may be made in a variety of other *Salmonella* genes including, but not limited to, *cya, crp, asd, cdt, phoP, phoQ, ompR*, outer membrane proteins, *dam, htrA* or other stress related genes, *aro, pur* and *gua.* As shown in the Examples, mutations in *aroA* and *htrA* were found to attenuate SE13A. The *aro* genes are enzymes involved in the shikimate biosynthesis pathway or the aromatase pathway and *aro* mutants are auxotrophic for the aromatic amino acids tryptophan, tyrosine and phenylalanine. *htrA* is a stress response gene that encodes a periplasmic protease that degrades aberrant proteins. Mutants in *htrA* are also attenuated and display increased sensitivity to hydrogen peroxide.

The mutations in *aroA* and *htrA* described in the Examples are deletion mutations, but the mutations can be made in a variety of ways. Suitably, the mutations are non-reverting mutations that cannot be repaired in a single step. Suitable mutations include deletions, inversions, insertions and substitutions. A vaccine vector may include more than one mutation, for example a vaccine vector may contain mutations in both *aroA* and *htrA.* Methods of making such mutations are well known in the art.

SE13A or the attenuated SE13A variants may be used as vaccine vectors. Polynucleotides encoding *fliC* polypeptide antigens and other antigens from any number of pathogenic organisms may be inserted into the bacteria and expressed by the bacteria to generate antigenic polypeptides. The polynucleotides may be inserted into the chromosome of the bacteria or encoded on plasmids or other extrachromosomal DNA. Polynucleotides encoding fliC antigens are inserted into a bacterial polynucleotide that is expressed in accordance with claim 1. Suitably, the bacterial polynucleotide encodes a transmembrane protein, and the polynucleotide encoding the fliC antigen is inserted into the bacterial polynucleotide sequence to allow expression of the fliC antigen on the surface of the bacteria. For example, the polynucleotide encoding *fliC* may be inserted in frame into the bacterial polynucleotide in a region encoding an external loop region of a transmembrane protein such that the bacterial polynucleotide sequence remains in frame. See Example 1.

Those of skill in the art will appreciate that the polynucleotide encoding the *fliC* antigen could be inserted in a wide variety of bacterial polynucleotides to provide expression and presentation of the *fliC* antigen to the immune cells of a subject treated with the bacterial vaccine vector. In the Examples, a polynucleotide encoding a *fliC* antigen was inserted into loop 9 of the *lamB* gene of SE13A. The polynucleotide encoding the *fliC* antigen may be included in a single copy or more than one copy. In the Examples, a bacterial vaccine vector containing a single copy of the *fliC* antigen inserted into loop 9 of *lamB* is described. Alternatively, copies of an epitope may be inserted into the bacterial vaccine vector at more than one location. The copies of the polynucleotide may be linked together or separated by a linker. Suitable linkers are known to those of skill in the art and include, but are not limited to a repeated amino acid, such as 1-10 serine residues.

The vaccine vector comprises a polynucleotide encoding a CD154 polypeptide that is capable of binding CD40 in the subject and stimulating the subject to respond to the vaccine vector and its associated antigen. As described above, these polynucleotides may be inserted into the chromosome of the vaccine vector or maintained extrachromosomally. The CD154 polynucleotide is inserted into a polynucleotide encoding an external portion of a transmembrane protein. The polynucleotide encoding a CD 154 polypeptide capable of enhancing the immune response to a foreign antigen may also encode the foreign antigen. The polynucleotide encoding a CD 154 polypeptide may be linked to the polynucleotide encoding the *fliC* antigen, such that in the vaccine vector the CD 154 polypeptide and the *fliC* antigen are present on the same polynucleotide. In the Examples, a polynucleotide encoding a polypeptide of CD154 that is capable of binding to CD40 also encodes the *fliC* antigen. See SEQ ID NOS: 1, 2, 9, 10 and 11 in the attached sequence listing. In the Examples, the polynucleotide encoding the *fliC* antigen and the polynucleotide encoding the CD 154 polypeptide are both inserted in loop 9 of the *lamB* gene. Those of skill in the art will appreciate that bacterial polynucleotides encoding other transmembrane proteins and other loops of the *lamB* gene may also be used.

The SE13A bacteria include an exogenous polynucleotide encoding a *fliC* polypeptide that is a portion of the full-length *fliC* polypeptide natively associated with *Salmonella.* Suitably a polynucleotide encoding a portion of the *fliC* polypeptide or the entire *fliC* polypeptide may be inserted into the vaccine vector. In the Examples, a seven amino acid polypeptide (SEQ ID NO:1) was incorporated into SE13A. Suitably, the portion of the *fliC* polypeptide inserted into the vaccine vector is an immunogenic fragment. An immunogenic fragment is a peptide or polypeptide capable of eliciting a cellular or humoral immune response. Suitably, an immunogenic fragment of *fliC* may be 6 or more amino acids, 10 or more amino acids, 15 or more amino acids or 20 or more amino acids of the full-length protein sequence.

Other suitable epitopes for inclusion in a *fliC* vaccine vector include, but are not limited to, polynucleotides encoding other bacterial polypeptides. One of skill in the art will appreciate that a variety of sequences may be used in combination with any other antigen and may also be used in conjunction with polypeptides encoding immune stimulatory peptides such as a polypeptide of CD154.

As discussed above, a polynucleotide encoding a CD154 polypeptide that is capable of enhancing the immune response to the antigen may be included in the vaccine vector. Suitably, the CD154 polypeptide is fewer than 50 amino acids long, more suitably fewer than 40, fewer than 30 or fewer than 20 amino acids in length. The polypeptide may be between 10 and 15 amino acids, between 10 and 20 amino acids or between 10 and 25 amino acids in length. The CD154 sequence and CD40 binding region are not highly conserved among the various species. The CD 154 amino acid sequences of chicken and human are provided in SEQ ID NO: 9 and SEQ ID NO: 8, respectively.

The CD40 binding regions of CD 154 have been determined for a number of species, including human, chicken, duck, mouse and cattle and are shown in SEQ ID NO: 3, SEQ ID NO: 4, SEQ ID NO:5, SEQ ID NO:6, SEQ ID NO:7, respectively. Although there is variability in the sequences in the CD40 binding region between species, the human CD154 polypeptide was able to enhance the immune response in chickens. Therefore, one may practice the teaching of the disclosure using species specific CD154 pplypeptides or a heterologous CD154 polypeptide.

In the Examples, several SE13A recombinant bacteria were generated. In each of the SE13A strains containing polynucleotides encoding both the *fliC* and CD154 peptides, the fliC polypepetide and the CD 154 polypeptide were encoded on the same polynucleotide and were in frame with each other and with the *Salmonella lamB* polynucleotide in which they were inserted. The CD154 polypeptide and the fliC polypeptide may be encoded by distinct polynucleotides. SE13A *aroA htrA fliC* contains a deletion in *aroA* and *htrA* and encodes both the *fliC* epitope (SEQ ID NO:1) and optionally the CD 154 polypeptide (SEQ ID NO: 3) inserted into loop 9 of *lamB.*

Compositions comprising an attenuated *Salmonella* strain and a pharmaceutically acceptable carrier are also provided. A pharmaceutically acceptable carrier is any carrier suitable for *in vivo* administration. Examples of pharmaceutically acceptable carriers suitable for use in the composition include, but are not limited to, water, buffered solutions, glucose solutions or bacterial culture fluids. Additional components of the compositions may suitably include, for example, excipients such as stabilizers, preservatives, diluents, emulsifiers and lubricants. Examples of pharmaceutically acceptable carriers or diluents include stabilizers such as carbohydrates (e.g., sorbitol, mannitol, starch, sucrose, glucose, dextran), proteins such as albumin or casein, protein-containing agents such as bovine serum or skimmed milk and buffers (e.g., phosphate buffer). Especially when such stabilizers are added to the compositions, the composition is suitable for freeze-drying or spray-drying.

Methods of enhancing immune responses in a subject by administering a vaccine vector containing a *fliC* polypeptide and a CD 154 polypeptide capable of binding to CD40 and activating CD40 are also provided. The vaccine vector comprising the polynucleotide encoding a CD154 polypeptide capable of binding to CD40 is administered to a subject in an amount effective to enhance the immune response of the subject to the vaccine. Suitably, the vaccine vector contains a polynucleotide encoding a polypeptide including amino acids 140-149 of the human CD154 polypeptide (SEQ ID NO: 8) or a homolog thereof. As noted above and demonstrated in the Examples, the CD40 binding region of CD154 is not conserved among species, yet heterologous CD154 was capable of enhancing the immune response (i.e. human sequence used in a chicken). Therefore, a homologue of amino acid 140-149 derived from one species may be used to stimulate an immune response in a distinct species.

Several suitable polypeptides are identified herein. Suitably, the polynucleotide encodes a CD154 polypeptide from the same species as the subject. Suitably, a polynucleotide encoding the polypeptide of SEQ ID NO: 3 is used in human subjects, a polynucleotide encoding the polypeptide of SEQ ID NO: 4 is used in chickens, a polynucleotide encoding the polypeptide of SEQ ID NO: 5 is used in ducks, a polynucleotide encoding the polypeptide of SEQ ID NO: 6 is used in mice, and a polynucleotide encoding the polypeptide of SEQ ID NO: 7 is used in cows. In the Examples, the human CD 154 polypeptide (SEQ ID NO: 3) was used in a chicken vaccine vector and was demonstrated to enhance the immune response to a foreign antigen. Thus other heterologous combinations of CD154 polypeptides and subjects may be useful in the methods of the invention. The CD154 polypeptide may be used to enhance the immune response in the subject to any foreign antigen or antigenic polypeptide present in the vaccine vector in addition to the *fliC* polypeptide. One of skill in the art will appreciate that the CD 154 polypeptide could be used to enhance the immune response to more than one antigenic polypeptide present in a vaccine vector.

The polypeptide from CD 154 stimulates an immune response at least in part by binding to its receptor, CD40. The Examples used a polypeptide homologous to the CD154 polypeptide which is expressed on immune cells of the subject and which is capable of binding to the CD40 receptor on macrophages and other antigen presenting cells. Binding of this ligand-receptor complex stimulates macrophage (and macrophage lineage cells such as dendritic cells) to enhance phagocytosis and antigen presentation while increasing cytokine secretions known to activate other local immune cells (such as B-lymphocytes). As such, molecules associated with the CD 154 peptide are preferentially targeted for immune response and expanded antibody production.

Potential vaccine vectors for use in the methods include, but are not limited to, *Salmonella* (*Salmonella enteritidis*), *Shigella, Escherichia (E. coli), Yersinia, Bordetella, Lactococcus, Lactobacillus, Bacillus, Streptococcus, Vibrio (Vibrio cholerae), Listeria,* adenovirus, poxvirus, herpesvirus, alphavirus, and adeno-associated virus.

In addition, methods of enhancing an immune response against flagellated bacteria and methods of reducing morbidity associated with subsequent infection with a flagellated bacterium are disclosed. Briefly, the methods comprise administering to a subject a vaccine vector comprising a polynucleotide sequence encoding a *fliC* polypeptide in an effective amount. The *fliC* polypeptides include SEQ ID NO:1-2 and 10-12. The insertion of the *fliC* polypeptides into the vaccine vector may be accomplished in a variety of ways known to those of skill in the art, including but not limited to, the scarless site-directed mutation system described in BMC Biotechnol. 2007 Sept, 17: 7(1): 59, Scarless and Site-directed Mutagenesis in Salmonella enteritidis chromosome. The vaccine vector may also be engineered to express the *fliC* polypeptides in conjunction with polynucleotides capable of enhancing the immune response as discussed above, such as in SEQ ID NO:8 and SEQ ID NO:9. In particular, a polypeptide of CD 154 capable of binding CD40 may be expressed by the vaccine vector to enhance the immune response of the subject to the fliC polypeptide. Optionally, the vaccine vector is a bacterium, such as *Salmonella, enteritidis.*

The useful dosage of the vaccine vector to be administered will vary depending on the age, weight and species of the subject, the mode and route of administration and the type of pathogen against which an immune response is sought. The composition may be administered in any dose sufficient to evoke an immune response. For bacterial vaccines, it is envisioned that doses ranging from 10³ to 10¹⁰ bacteria, from 10⁴ to 10⁹ bacteria, or from 10⁵ to 10⁷ bacteria are suitable. The composition may be administered only once or may be administered two or more times to increase the immune response. For example, the composition may be administered two or more times separated by one week, two weeks, or by three or more weeks. The bacteria are suitably viable prior to administration, but in some embodiments the bacteria may be killed prior to administration. In some embodiments, the bacteria may be able to replicate in the subject, while in other embodiments the bacteria may not be capable of replicating in the subject.

For administration to animals or humans, the compositions may be administered by a variety of means including, but not limited to, intranasally, mucosally, by spraying, intradermally, parenterally, subcutaneously, orally, by aerosol, or intramuscularly. Eye-drop administration or addition to drinking water or food are additionally suitable. For chickens, the compositions may be administered in ovo.

Also described herein are methods of enhancing immune responses in a subject. Suitable subjects may include, but are not limited to, vertebrates, suitably mammals, suitably a human, and birds, suitably poultry such as chickens. Other animal models of infection may also be used. Enhancing an immune response includes, but is not limited to, inducing a therapeutic or prophylactic effect that is mediated by the immune system of the subject. Specifically, enhancing an immune response may include enhanced production of antibodies, enhanced class switching of antibody heavy chains, maturation of antigen presenting cells, stimulation of helper T cells, stimulation of cytolytic T cells or induction of T and B cell memory.

It is envisioned that several epitopes or antigens from the same or different pathogens may be administered in combination in a single vaccine vector to generate an enhanced immune response against multiple antigens. Recombinant vaccine vectors may encode antigens from multiple pathogenic microorganisms, viruses or tumor associated antigens. Administration of vaccine vectors capable of expressing multiple antigens has the advantage of inducing immunity against two or more diseases at the same time. For example, live attenuated bacteria, such as *Salmonella enteritidis* 13A, provide a suitable vaccine vector for eliciting an immune response against multiple antigens.

Bacterial vaccines may be constructed using exogenous polynucleotides encoding antigens which may be inserted into the bacterial genome at any non-essential site or alternatively may be carried on a plasmid using methods well known in the art. One suitable site for insertion of polynucleotides is within external portions of transmembrane proteins or coupled to sequences that target the exogenous polynucleotide for secretory pathways. One example of a suitable transmembrane protein for insertion of polynucleotides is the *lamB* gene. In the Examples, fliC and CD154 polynucleotides were inserted into loop 9 of the *lamB* sequence.

Exogenous polynucleotides include, but are not limited to, polynucleotides encoding antigens selected from pathogenic microorganisms or viruses and include polynucleotides of the vaccine vector which are expressed in such a way that an effective immune response is generated. Such polynucleotides may be derived from pathogenic viruses such as influenza (e.g., M2e, hemagglutinin, or neuraminidase), herpesviruses (e.g., the genes encoding the structural proteins of herpesviruses), retroviruses (e.g., the gp160 envelope protein), adenoviruses, paramyxoviruses, coronaviruses and the like. Exogenous polynucleotides can also be obtained from pathogenic bacteria, e.g., genes encoding bacterial proteins such as toxins, and outer membrane proteins. Further, exogenous polynucleotides from parasites are attractive candidates for use of a vector vaccine.

Polynucleotides encoding polypeptides involved in triggering the immune system may also be included in a vaccine vector, such as a live attenuated *Salmonella* vaccine. The polynucleotides may encode immune system molecules known for their stimulatory effects, such as an interleukin, Tumor Necrosis Factor, or an interferon, or another polynucleotide involved in immune-regulation. The vaccine vector may also include polynucleotides encoding peptides known to stimulate an immune response, such as the CD 154 polypeptide described herein.

The following examples are meant only to be illustrative.

### EXAMPLES

### Example 1. Construction of fliC and fliC/CD154 inserts.

### Strains and Culture Conditions

All plasmids were first maintained in TOP10 *E. coli* cells (Invitrogen, Carlsbad, CA, USA) unless described otherwise. *Salmonella enteritidis* 13A was used for introduction of mutations. *Salmonella enteritidis* strain 13A was a field isolate available from USDA/APHIS/NVSL and deposited with the ATCC as deposit number PTA-7871. Bacteria carrying plasmid pKD46 were grown at 30°C. Other bacteria were grown at 37°C. Plasmid curing was conducted at 37°C.

Luria-Bertani (LB) media was used for routine growth of cells, and SOC media (Invitrogen, Carlsbad, CA, USA) was used for phenotypic expression after electroporation. When appropriate, the following antibiotics were added to the media: ampicillin (Amp) at 100µg/ml, kanamycin (Km) at 50µg/ml, and chloramphenicol (Cm) at 25µg/ml.

### Plasmids

Plasmids pKD46, pKD13, and pBC-I-SceI were described previously (Datsenko and Wanner, PNAS 2000, 97:6640-6645 and Kang et al., J Bacteriol 2004, 186:4921-4930). Plasmid pKD46 encodes Red recombinase enzymes which mediate homologous recombination of incoming linear DNA with chromosomal DNA. This plasmid also contains the Ampicillin resistance gene and is temperature-sensitive so that it requires 30°C for maintenance in the cell. Plasmid pKD13 served as a template for amplification of the Km resistance (Km^{r}) gene used in overlapping PCR. Plasmid pBC-I-SceI, which is maintained in the cell at 37°C, produces the I-SceI enzyme, which cleaves the following 18 base pair, rare recognition sequence: 5'-TAGGGATAACAGGGTAAT-3' (SEQ ID NO:13). Plasmid pBC-I-SceI also contains the chloramphenicol resistance (Cm^{r}) gene.

### PCR

All primers used for PCR are listed in Table 1. Typically, PCR was performed using approximately 0.1µg of purified genomic, plasmid or PCR-generated DNA (Qiagen, Valencia, CA, USA), 1x cloned *Pfu* polymerase buffer, 5U *Pfu* polymerase (Stratagene La Jolla, CA, USA), 1mM dNTPs (GE Healthcare Bio-Sciences Corp., Piscataway, NJ), and 1.2µM of each primer in a total volume of 50 µL. The DNA engine thermal cycler (Bio-Rad, Hercules, CA, USA) was used with the following amplification conditions: 94°C for 2 minutes; 30 cycles of 94°C sec for 30 sec, 58°C for 60 sec, 72°C for 90 sec per 1 kb; and 72°C for 10 minutes for final extension. Each PCR product was gel purified (Qiagen, Valencia, CA, USA) and either eluted in 25µL EB buffer for preparation of templates used in overlapping extension PCR or in 50µL EB buffer, ethanol precipitated and suspended in 5µL of ddH₂O for electroporation into *S. enteritidis.*

**Table 1. Primer sequences**

| **Primer** | **Amplified region** | **Primer sequence** |
|---|---|---|
| lam-up-f | loop 9 up | 5'TGTACAAGTGGACGCCAATC 3' (SEQ ID NO: 14) |
| lam-up-f | | *5'GTTATCGCCGTCTTTGATATAGCC* 3' (SEQ ID NO: 15) |
| lam-dn-f | looop 9 dn | *5'ATTTCCCGTTATGCCGCAGC* 3' (SEQ ID NO: 16) |
| lam-dn-f | | 5'GTTAAACAGAGGGCGACGAG 3' (SEQ ID NO: 17) |
| Km-f | I-SceI/Km^{r} gene | |
| Km-r | | |
| Kan4f | inside Km^{r} gene: sequencing | 5'CAAAAGCGCTCTGAAGTTCC 3' (SEQ ID NO: 20) |
| Kan4r | | 5'GCGTGAGGGGATCTTGAAGT 3' (SEQ ID NO: 21) |
| fliC up reverse | fliC/ loop 9 up | |
| fliC down forward | fliC/ loop 9 down | |
| fliC hCD154 up reverse | fliC-hCD154/ loop 9 up | |
| fliC hCD154 up reverse | fliC-hCD154/ loop 9 down | |
| fliC cCD154 up reverse | fliC-cCD 154/ loop 9 up | |
| fliC cCD154 up reverse | fliC-cCD154/ loop 9 down | |
| lam 3f | outer regions of loop 9: sequencing | 5'GCCATCTGCTTGGTGATAA 3' (SEQ ID NO: 28) |
| lam 3r | | 5'CGCTGGTATTTTGCGGTACA 3' (SEQ ID NO: 28) |

In Table 1, italicized nucleotides are complementary to either side of the *lamB* gene loop 9 insertion site, which corresponds to nucleotide 1257 using *S. typhimurium* as an annotated reference genome. Bold font nucleotides represent the I-SceI recognition site in the Km-f primer.

### Electroporation

Transformation of pKD46 into *S. enteritidis* was the first step carried out so that Red recombinase enzymes could be used for mediating recombination of subsequent mutations. Plasmid pKD46 was harvested from *E. coli* BW25113 (Datsenko and Wanner, PNAS 2000, 97:6640-6645) using a plasmid preparation kit (Qiagen Valencia, CA, USA). Then 0.5µL of pKD46 DNA was used for transformation into *S. enteritidis* 13A which had been prepared for electroporation. (Datsenko and Wanner, PNAS 2000, 97:6640-6645). Briefly, cells were inoculated into 10-15mL of 2X YT broth and grown at 37°C overnight. Then 100µL of overnight culture was re-inoculated into 10mL fresh 2X YT broth at 37°C for 3-4 hours. Cells to be transformed with pKD46 plasmid were heated at 50°C for 25 minutes to help inactivate host restriction. Cells were washed five times in ddH₂O water and resuspended in 60µL of 10% glycerol. Cells were then pulsed at 2400-2450kV for 1-6ms, incubated in SOC for 2-3 hours at 30°C and plated on LB media with appropriate antibiotics. *S. enteritidis* transformants with pKD46 were maintained at 30°C. When these transformants were prepared for additional electroporation reactions, all steps were the same except that 15% arabinose was added to induce Red recombinase enzymes one hour prior to washing, and cells did not undergo the 50°C heat step.

### Loop 9 up- I-SceI/Km^{r}-Loop 9 down Construct

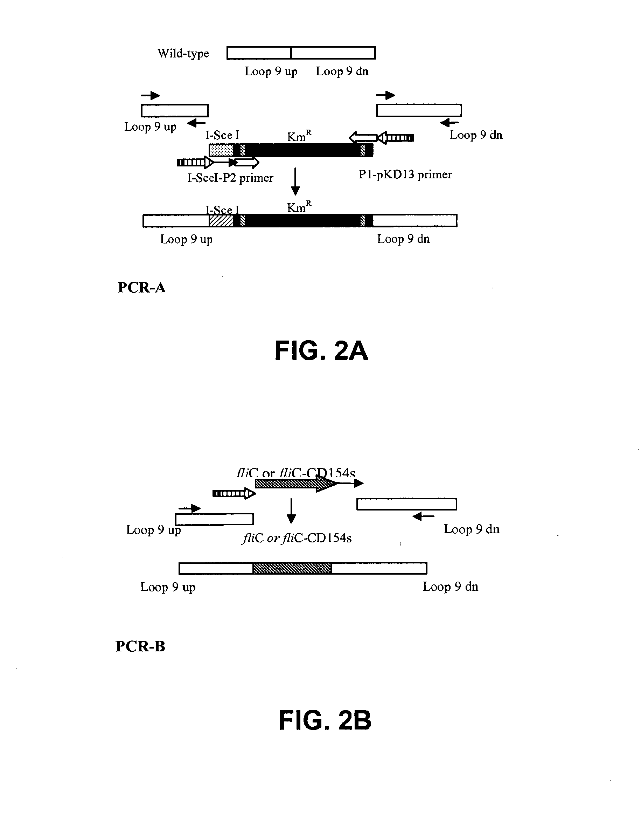

Introduction of I-SceI enzyme recognition site along with the Km^{r} gene into loop 9 of the *lamB* gene was done by combining the Red recombinase system (Datsenko and Wanner, PNAS 2000, 97:6640-6645) and overlapping PCR (Horton et al., BioTechniques 1990, 8:528-535). The insertion site corresponds to nucleotide 1257 of the *lamB* gene using *Salmonella typhimurium* LT2 (S. *typhimurium*) as an annotated reference genome. First, the upstream and downstream regions immediately flanking the loop 9 insertion site (loop 9 up and loop 9 down, respectively) were amplified separately. Primers used were lam-up-f and lam-up-r for loop 9 up and lam-dn-f and lam-dn-r for loop 9 down. Then the Km^{r} gene from pKD13 plasmid was amplified using primers Km-f and Km-r. Here, the I-SceI enzyme site was synthetically added to the 5' end of Km-f primer then preceded by a region complimentary to the loop-up-r primer. Likewise, a region complimentary to the loop-dn-f primer was added to the 5' end of Km-r primer. The complimentary regions allow all 3 PCR products to anneal when used as templates in one PCR reaction. Figure 2a represents this design scheme. PCR fragments consisting of loop 9 up- I-SceI/ Km^{r}- loop 9 down sequence (PCR-A) were electroporated into *S. enteritidis* cells, which harbored pKD46 and were induced by arabinose, and then plated on LB with Km plates. To verify the correct sequence orientation of the mutation, we performed colony PCR with primer pairs Kan4F/lam3f and Kan4R/lam3r, where Kan4F and Kan4R are Km^{r} gene-specific primers and lam3f and lam3r are primers located outside the *lamB* loop 9 region. These PCR fragments were gel purified (Qiagen, Valencia, CA, USA) and used for DNA sequencing.

### Loop 9 up- fliC or CD154s or combination sequence- Loop 9 down Construct

The final overlapping PCR fragment, PCR-B, contained the added fliC antigen (or combination with CD154 sequences flanked by loop 9 up and down regions (Figure 2b). Combination sequences consisted of *fliC* polynucleotide comprising SEQ ID NO:1 and a CD154 polynucleotide sequence along with spacers such as Glycine (Gly) or Serine (Ser) residues.

To shorten the amount of steps for construction of the next fragment, the *fliC* or *fliC-*CD154 sequence was synthetically added to the 5' end of the lam-dn-f primer and preceded by the complimentary region to the loop-up-r primer. The previously used PCR product for loop 9 up could be used together with the newly constructed PCR product in which *fliC* or *fliC*-CD154 were incorporated at the 5' end of loop 9 down to perform the final PCR reaction. However, for other insert sequences (referred to as combination sequences), an extra PCR step was needed, due to the longer lengths of insert sequences, to amplify loop 9 up with added nucleotides specific to insertion sequences connected to loop-up-r primer. The coding sequence for Gly (GGT) and Serine (TCC) as well as all other amino acids were chosen based on compiled data of the most frequently used codons in *E. coli* and *Salmonella typhimurium* proteins. See Table 1 for further details of primer design.

### Genomic Replacement of I-SceI/ Km^{r} with fliC or fliC-CD154

PCR-B products were electroporated into *S. enteritidis* cells along with plasmid pBC-I-SceI at a molar ratio of approximately 40:1 (Kang et al., J Bacteriol 2004, 186:4921-4930). Clones for each PCR-B recombination mutation were chosen according to the ability to grow on Cm plates but not on Km plates, due to the replacement of PCR-B for the Km^{r} encoding PCR-A sequence. Modified regions in the selected clones were PCR-amplified, and DNA sequences were determined using primers lam3f and lam3r located outside the loop 9 down and up amplified regions.

### I-SceI site/ Km^{r} insertion mutation

The first mutation step involved designing a PCR fragment, PCR-A, which would serve as the carrier of the I-SceI site/ Km^{r} cassette to be inserted into the *lamB* site. PCR-A consisted of the I-SceI enzyme recognition site adjacent to the Km^{r} gene with approximately 200- 300bp of flanking DNA on each end homologous to the upstream and downstream regions of *lamB* loop 9 insertion site (loop 9 up and loop 9 down, respectively). The fragment was introduced into *S. enteritidis* cells expressing Red recombinase enzymes and Km^{r} colonies were selected. After screening a few colonies by colony PCR, positive clones were sequenced for the desired inserted I-SceI site/ Km^{r} sequence, and the identified mutant was selected and designated as SE164.

### Genomic Replacement of I-Scel/ Km^{r} with fliC or fliC-CD154

The second mutation step required constructing a PCR fragment, referred to as PCR-B and shown in Figure 2B, consisting of the final insertion sequence, *fliC* or *fliC*-CD154, flanked by *lamB* homologous fragments. PCR-B amplicons have no selection marker and must be counter-selected after replacement for the previous I-Scel site/ Km^{r} mutation in SE164. Plasmid pBC-I-SceI encodes the Cm^{r} gene and the I-SceI enzyme, which will cut the genome at the I-SceI site of SE164. Therefore, pBC-I-SceI was electroporated into SE164 along with PCR-B. After recombination of PCR-B to replace PCR-A, positive clones were chosen based on the ability to grow on Cm but not on Km. After DNA sequencing of mutants to confirm successful recombination of PCR-B, the strains were designated *fliC, fliC-*cCD154 and *fliC*-hCD154. Ten random clones for each the *fliC* and *fliC*-CD154 insertion were used for PCR with lam 3f and lam 3r then digested using unique restriction enzymes sites for each insertion sequence and 100% of clones tested by digestion were positive for the desired mutation sequence. Sequencing results demonstrated that the insertion of *fliC* or *fliC-*CD 154 was exactly into the loop 9 region without the addition of extraneous nucleotides.

### Example 2. Attenuation of fliC or fliC-CD154 mutants/inserts.

Attenuation of SE13A was achieved by deletion mutation of the *aroA* gene and/or the *htrA* gene. Mutation of the *aroA* gene, a key gene in the chorismic acid pathway of bacteria, results in a severe metabolic deficiency which affects seven separate biochemical pathways. Mutation of the *htrA* gene reduces the cell's ability to withstand exposure to low and high temperatures, low pH, and oxidative and DNA damaging agents and reduces the bacteria's virulence.

To achieve deletion mutations in SE13A, the target gene sequence in the bacterial genome of *S. enteritidis* was replaced with the Km resistant gene sequence. This was completed using overlapping extension PCR and electroporation of the PCR products as described above. The Km resistance gene was targeted into the genomic region containing the genes of interest (*aroA* or *htrA*) by flanking the Km resistance gene with 200-300 base pairs of sequences homologous to the genes of interest. Once Km resistant mutants were obtained, the *aroA* or *htrA* deletion mutations were confirmed by DNA sequencing. The attenuated strains were also tested *in vivo* with regards to clearance time. Both of the attenuated strains had quicker clearance times than did the wildtype 13A strain, but both were able to colonize the liver, spleen, and cecal tonsils of chickens after oral infection. In addition, an attenuated strain lacking both *aroA* and *htrA* was also isolated.

### Example 3. Colonization of chickens

Day-of-hatch chicks (40 per group) were inoculated with about 1 x 10⁸ cfu of various Salmonella isolates or saline control. The Salmonella isolates included the following: wtSE represents the original field isolate of *Salmonella enteritidis* 13A (SE13A); fliC represents the double attenuated (i.e., aroA and htrA) wild type SE13A expressing fliC in the lamB loop (cell surface); fliC-CD154C represents the double attenuated wild type SE13A similarly expressing fliC and a chicken CD 154 oligopeptide.

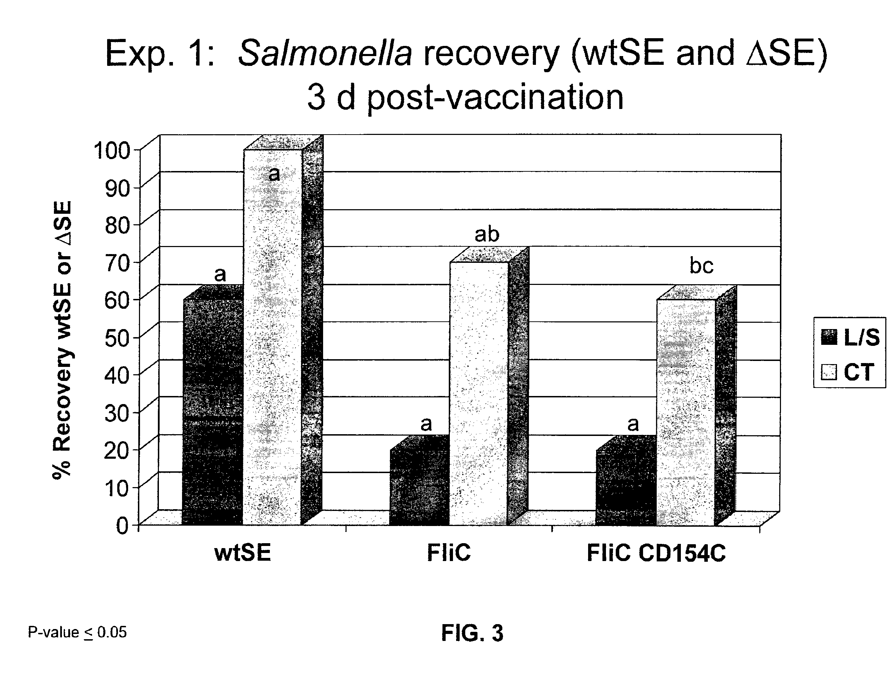

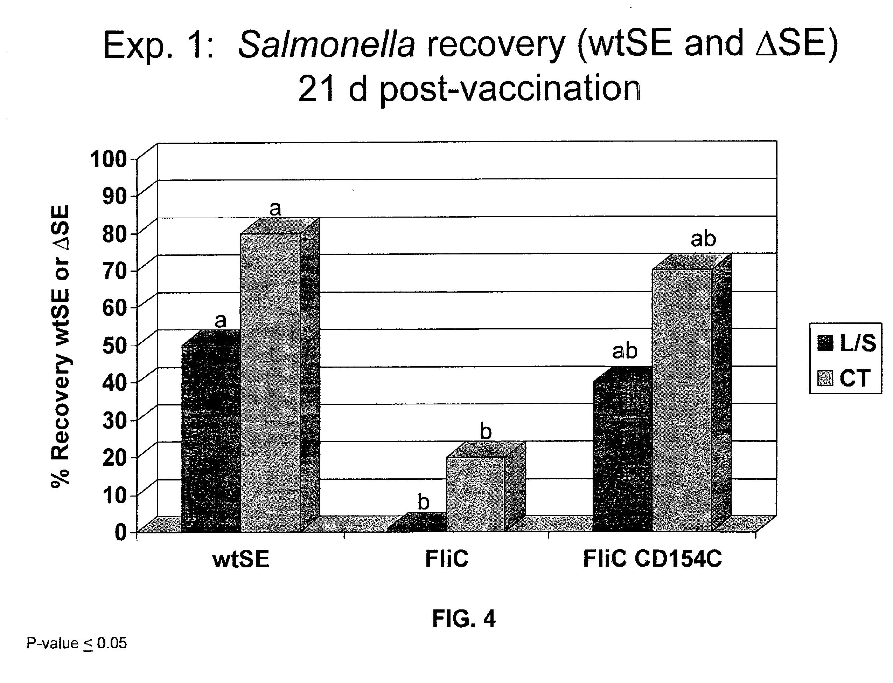

On days 3 and 21 post-vaccination the cecal tonsils (CT) and the liver and spleen (L/S) were harvested and bacterial recovery assessed by a standard colony forming unit (cfu) assay in 10 animals per group. The results are shown in Figure 3 (Day 3) and Figure 4 (Day 21) and are reported as Percent wtSE recovery as measured by the number of cfu recovered compared to the inoculum. No (zero) Salmonellae were recovered from either the non-challenged controls (control) at day 3 or day 21 post-vaccination or the chicks inoculated with the fliC mutant by day 21 post-vaccination in the liver or spleen. These results suggest that the chicks are mounting an effective immune response against the *fliC* antigen that allows them to clear the vaccine strain more rapidly than the wild-type SE13A. Different letters on the top of the bars in the graphs are indicative of significant differences from the wtSE (p<0.05). Therefore expression of *fliC* polypeptide on the surface of the bacterium induces a more robust immune response. Similar results were obtained when a human CD 154 oligopeptide was used in place of the chicken CD 154 peptide.

Serum was collected from the inoculated chicks and *fliC* specific antibody was measured by ELISA using standard procedures. *FliC* antibody production was not significantly increased in the animals vaccinated with *fliC* or *fliC*-CD154 containing SE.

## Claims

1. A bacterial vaccine comprising a first polynucleotide sequence encoding a *fliC* polypeptide and a second polynucleotide sequence encoding a CD154 polypeptide capable of binding CD40, the CD154 polypeptide having fewer than 50 amino acids and comprising amino acids 140-149 of SEQ ID NO: 8, or at least one of SEQ ID NO: 3, SEQ ID NO: 4, SEQ ID NO: 5, SEQ ID NO: 6, or SEQ ID NO: 7, wherein the *fliC* polypeptide consists of SEQ ID NO: 1, SEQ ID NO: 2, SEQ ID NO: 10, SEQ ID NO: 11, SEQ ID NO: 12, an immunogenic fragment of SEQ ID NO: 1, an immunogenic fragment of SEQ ID NO: 2, an immunogenic fragment of SEQ ID NO: 10, an immunogenic fragment of SEQ ID NO: 11, or an immunogenic fragment of SEQ ID NO: 12; and wherein the first polynucleotide sequence and the second polynucleotide sequence are inserted into a third polynucleotide sequence encoding an external portion of a transmembrane protein.

2. The vaccine of claim 1, wherein the bacterium is selected from the group consisting of Salmonella species, Escherichia species, Bacillus species and Lactobacillus species.

3. The vaccine of any of the preceding claims, wherein the vaccine comprises more than one copy of the first polynucleotide sequence, more than one copy of the second polynucleotide sequence or more than one copy of first polynucleotide sequence and more than one copy of the second polynucleotide sequence.

4. The vaccine of any of the preceding claims, wherein the first polynucleotide sequence is linked in frame to the second polynucleotide sequence.

5. The vaccine of any of the preceding claims, wherein the first polynucleotide is an exogenous polynucleotide.

6. The vaccine of any of the preceding claims, wherein the bacterium is a Salmonella.

7. The vaccine of any of the preceding claims, wherein the transmembrane protein is LamB.

8. A vaccine comprising a variant of *Salmonella enteritidis* 13A, wherein the Salmonella comprises an exogenous first polynucleotide sequence encoding a *fliC* polypeptide and a second polynucleotide sequence encoding a CD154 polypeptide capable of binding CD40, the CD154 polypeptide having fewer than 50 amino acids and comprising amino acids 140-149 of SEQ ID NO: 8, or at least one of SEQ ID NO: 3, SEQ ID NO: 4, SEQ ID NO: 5, SEQ ID NO: 6, or SEQ ID NO: 7, wherein the *fliC* polypeptide consists of SEQ ID NO: 1, SEQ ID NO: 2, SEQ ID NO: 10, SEQ ID NO: 11, SEQ ID NO: 12, an immunogenic fragment of SEQ ID NO: 1, an immunogenic fragment of SEQ ID NO: 2, an immunogenic fragment of SEQ ID NO: 10, an immunogenic fragment of SEQ ID NO: 11, or an immunogenic fragment of SEQ ID NO: 12; and wherein the first polynucleotide sequence and the second polynucleotide sequence are inserted into a third polynucleotide sequence encoding an external portion of a transmembrane protein.

9. The vaccine of claim 8, wherein the transmembrane protein is lamB.

10. The vaccine of any one of claims 8-9, wherein the first polynucleotide sequence is linked in frame to the second polynucleotide sequence.

11. The vaccine of any one of claims 8-10, wherein the vaccine comprises more than one copy of the first polynucleotide sequence, more than one copy of the second polynucleotide sequence or more than one copy of first polynucleotide sequence and more than one copy of the second polynucleotide sequence.

12. The vaccine of any one of the preceding claims for use in a method of enhancing an immune response against a flagellated bacterium.

13. The vaccine of claim 12, wherein the vaccine comprises a killed bacterium.

14. The vaccine of any one of claims 12-13, wherein the vaccine comprises a bacterium not capable of replicating.

## Patentansprüche

1. Bakterielle Vakzine, die eine erste Polynucleotidsequenz, die für ein fliC-Polypeptid kodiert, und eine zweite Polynucleotidsequenz, die für ein CD154-Polypeptid kodiert, das in der Lage ist, CD40 zu binden, umfasst, wobei das CD154-Polypeptid weniger als 50 Aminosäuren aufweist und die Aminosäuren 140 bis 149 von Seq.-ID Nr. 8 oder zumindest eine von Seq.-ID Nr. 3, Seq.-ID Nr. 4, Seq.-ID Nr. 5, Seq.-ID Nr. 6 oder Seq.-ID Nr. 7 umfasst, worin das fliC-Polypeptid aus Seq.-ID Nr. 1, Seq.-ID Nr. 2, Seq.-ID Nr. 10, Seq.-ID Nr. 11, Seq.-ID Nr. 12, einem immunogenen Fragment von Seq.-ID Nr. 1, einem immunogenen Fragment von Seq.-ID Nr. 2, einem immunogenen Fragment von Seq.-ID Nr. 10, einem immunogenen Fragment von Seq.-ID Nr. 11 oder einem immunogenen Fragment von Seq.-ID Nr. 12 besteht; und worin die erste Polynucleotidsequenz und die zweite Polynucleotidsequenz in eine dritte Polynucleotidsequenz insertiert sind, die für einen externen Teil eines Transmembranproteins kodiert.

2. Vakzine nach Anspruch 1, worin das Bakterium aus der aus Salmonella-Spezies, Escherichia-Spezies, Bacillus-Spezies und Lactobacillus-Spezies bestehenden Gruppe ausgewählt ist.

3. Vakzine nach einem der vorangegangenen Ansprüche, worin die Vakzine mehr als eine Kopie der ersten Polynucleotidsequenz, mehr als eine Kopie der zweiten Polynucleotidsequenz oder mehr als eine Kopie der ersten Polynucleotidsequenz und mehr als eine Kopie der zweiten Polynucleotidsequenz umfasst.

4. Vakzine nach einem der vorangegangenen Ansprüche, worin die erste Polynucleotidsequenz im Raster an die zweite Polynucleotidsequenz gebunden ist.

5. Vakzine nach einem der vorangegangenen Ansprüche, worin das erste Polynucleotid ein exogenes Polynucleotid ist.

6. Vakzine nach einem der vorangegangenen Ansprüche, worin das Bakterium eine Salmonelle ist.

7. Vakzine nach einem der vorangegangenen Ansprüche, worin das Transmembranprotein LamB ist.

8. Vakzine, die eine Variante von Salmonella enteritidis 13A umfasst, worin die Salmonelle eine exogene erste Polynucleotidsequenz, die für ein fliC-Polypeptid kodiert, und eine zweite Polynucleotidsequenz, die für ein CD154-Polypeptid kodiert, das in der Lage ist, CD40 zu binden, umfasst, wobei das CD154-Polypeptid weniger als 50 Aminosäuren aufweist und die Aminosäuren 140 bis 149 von Seq.-ID Nr. 8 oder zumindest eine von Seq.-ID Nr. 3, Seq.-ID Nr. 4, Seq.-ID Nr. 5, Seq.-ID Nr. 6 oder Seq.-ID Nr. 7 umfasst, worin das fliC-Polypeptid aus Seq.-ID Nr. 1, Seq.-ID Nr. 2, Seq.-ID Nr. 10, Seq.-ID Nr. 11, Seq.-ID Nr. 12, einem immunogenen Fragment von Seq.-ID Nr. 1, einem immunogenen Fragment von Seq.-ID Nr. 2, einem immunogenen Fragment von Seq.-ID Nr. 10, einem immunogenen Fragment von Seq.-ID Nr. 11 oder einem immunogenen Fragment von Seq.-ID Nr. 12 besteht; und worin die erste Polynucleotidsequenz und die zweite Polynucleotidsequenz in eine dritte Polynucleotidsequenz insertiert sind, die für einen externen Teil eines Transmembranproteins kodiert.

9. Vakzine nach Anspruch 8, worin das Transmembranprotein lamB ist.

10. Vakzine nach einem der Ansprüche 8 und 9, worin die erste Polynucleotidsequenz im Raster an die zweite Polynucleotidsequenz gebunden ist.

11. Vakzine nach einem der Ansprüche 8 bis 10, worin Vakzine mehr als eine Kopie der ersten Polynucleotidsequenz, mehr als eine Kopie der zweiten Polynucleotidsequenz oder mehr als eine Kopie der ersten Polynucleotidsequenz und mehr als eine Kopie der zweiten Polynucleotidsequenz umfasst.

12. Vakzine nach einem der vorangegangenen Ansprüche zur Verwendung in einem Verfahren zur Verstärkung einer Immunantwort gegen ein flagelliertes Bakterium.

13. Vakzine nach Anspruch 12, worin die Vakzine ein getötetes Bakterium umfasst.

14. Vakzine nach einem der Ansprüche 12 und 13, worin die Vakzine ein Bakterium umfasst, das nicht zur Replikation in der Lage ist.

## Revendications

1. Vaccin bactérien comprenant une première séquence polynucléotidique codant pour un polypeptide *fliC* et une deuxième séquence polynucléotidique codant pour un polypeptide CD154 capable de se lier à CD40, le polypeptide CD154 possédant moins de 50 acides aminés et comprenant les acides aminés 140-149 de SEQ ID NO: 8, ou au moins l'une de SEQ ID NO: 3, SEQ ID NO: 4, SEQ ID NO: 5, SEQ ID NO: 6, ou SEQ ID NO: 7, où le polypeptide *fliC* consiste en SEQ ID NO: 1, SEQ ID NO: 2, SEQ ID NO: 10, SEQ ID NO: 11, SEQ ID NO: 12, un fragment immunogène de SEQ ID NO: 1, un fragment immunogène de SEQ ID NO: 2, un fragment immunogène de SEQ ID NO: 10, un fragment immunogène de SEQ ID NO: 11, ou un fragment immunogène de SEQ ID NO: 12 ; et où la première séquence polynucléotidique et la deuxième séquence polynucléotidique sont insérées dans une troisième séquence polynucléotidique codant pour une partie externe d'une protéine transmembranaire.

2. Vaccin selon la revendication 1, dans lequel la bactérie est sélectionnée dans le groupe consistant en l'espèce Salmonella, l'espèce Escherichia, l'espèce Bacillus et l'espèce Lactobacillus.

3. Vaccin selon l'une quelconque des revendications précédentes, où le vaccin comprend plus d'une copie de la première séquence polynucléotidique, plus d'une copie de la deuxième séquence polynucléotidique ou plus d'une copie de la première séquence polynucléotidique et plus d'une copie de la deuxième séquence polynucléotidique.

4. Vaccin selon l'une quelconque des revendications précédentes, dans lequel la première séquence polynucléotidique est liée dans le cadre à la deuxième séquence polynucléotidique.

5. Vaccin selon l'une quelconque des revendications précédentes, dans lequel le premier polynucléotide est un polynucléotide exogène.

6. Vaccin selon l'une quelconque des revendications précédentes, dans lequel la bactérie est une Salmonella.

7. Vaccin selon l'une quelconque des revendications précédentes, dans lequel la protéine transmembranaire est LamB.

8. Vaccin comprenant un variant de *Salmonella enteritidis* 13A, où la Salmonella comprend une première séquence polynucléotidique exogène codant pour un polypeptide *fliC* et une deuxième séquence polynucléotidique codant pour un polypeptide CD154 capable de se lier à CD40, le polypeptide CD154 possédant moins de 50 acides aminés et comprenant les acides aminés 140-149 de SEQ ID NO: 8, ou au moins l'une de SEQ ID NO: 3, SEQ ID NO: 4, SEQ ID NO: 5, SEQ ID NO: 6, ou SEQ ID NO: 7, où le polypeptide *fliC* consiste en SEQ ID NO: 1, SEQ ID NO: 2, SEQ ID NO: 10, SEQ ID NO: 11, SEQ ID NO: 12, un fragment immunogène de SEQ ID NO: 1, un fragment immunogène de SEQ ID NO: 2, un fragment immunogène de SEQ ID NO: 10, un fragment immunogène de SEQ ID NO: 11, ou un fragment immunogène de SEQ ID NO: 12 ; et où la première séquence polynucléotidique et la deuxième séquence polynucléotidique sont insérées dans une troisième séquence polynucléotidique codant pour une partie externe d'une protéine transmembranaire.

9. Vaccin selon la revendication 8, dans lequel la protéine transmembranaire est LamB.

10. Vaccin selon l'une quelconque des revendications 8 à 9, dans lequel la première séquence polynucléotidique est liée dans le cadre à la deuxième séquence polynucléotidique.

11. Vaccin selon l'une quelconque des revendications 8 à 10, où le vaccin comprend plus d'une copie de la première séquence polynucléotidique, plus d'une copie de la deuxième séquence polynucléotidique ou plus d'une copie de la première séquence polynucléotidique et plus d'une copie de la deuxième séquence polynucléotidique.

12. Vaccin selon l'une quelconque des revendications précédentes destiné à être utilisé dans un procédé de stimulation d'une réponse immunitaire contre une bactérie flagellée.

13. Vaccin selon la revendication 12, où le vaccin comprend une bactérie tuée.

14. Vaccin selon l'une quelconque des revendications 12 à 13, où le vaccin comprend une bactérie incapable de se répliquer.
